(19) Europäisches Patentamt
European Patent Office
Office européen des brevets

(11) Veröffentlichungsnummer: **0 419 918 A2**

(12) **EUROPÄISCHE PATENTANMELDUNG**

(21) Anmeldenummer: **90117346.8**

(22) Anmeldetag: **08.09.90**

(51) Int. Cl.5: **C07D 285/12**, C07D 271/10, A61K 31/41

(30) Priorität: **23.09.89 DE 3931843**

(43) Veröffentlichungstag der Anmeldung: **03.04.91 Patentblatt 91/14**

(84) Benannte Vertragsstaaten: **AT BE CH DE DK ES FR GB GR IT LI NL SE**

(71) Anmelder: **BAYER AG**

**W-5090 Leverkusen 1 Bayerwerk(DE)**

(72) Erfinder: **Bonse, Gerhard, Dr.**
**Wolfskaul 3**
**W-5000 Köln 80(DE)**
Erfinder: **Müller, Nikolaus, Dr.**
**Knipprather Strasse 98**
**W-4019 Monheim(DE)**
Erfinder: **Harder, Achim, Dr. Dr.**
**Auf dem Bruch 49**
**W-5090 Leverkusen 3(DE)**

(54) **Substituierte 1,3,4-Oxa(thia)-diazolinone Verfahren zu ihrer Herstellung und ihre Verwendung bei der Bekämpfung von Endoparasiten.**

(57) Die vorliegende Erfindung betrifft neue substituierte 1,3,4-Oxa- und Thiadiazolinone der Formel (I)

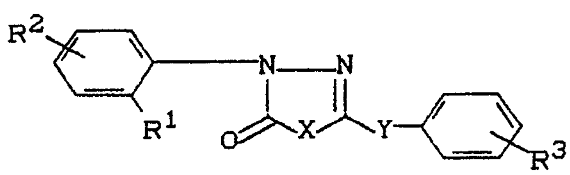

(I)

in welcher
$R^1$ für Wasserstoff, Alkyl, Alkoxy, Alkylthio, Halogen, Halogenalkyl steht,
$R^2$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxyl Arylthio, die ihrerseits wieder substituiert sein können, steht,
$R^3$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, NO, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
X für O oder S steht,
Y für O oder S steht,
ausgenommen die Verbindungen 5-Phenoxy-3-(3,4-dichlorphenyl)-1,3,4-oxadiazol-2-(3H)-on, 5-Phenoxy-3-(3-chlor-4-fluorphenyl)-1,3,4-oxadiazolo-2-(3H)on.

Die Verbindungen sind endoparasitizid wirksam.

EP 0 419 918 A2

## SUBSTITUIERTE 1,3,4-OXA(THIA)DIAZOLINONE VERFAHREN ZU IHRER HERSTELLUNG UND IHRE VERWENDUNG DER BEKÄMPFUNG VON ENDOPARASITEN

Die vorliegende Erfindung betrifft neue substituierte 1,3,4-Oxa(thia)diazolinone, Verfahren zu ihrer Herstellung und ihre Verwendung zur Bekämpfung von Endoparasiten.

Substituierte 2-Alkoxy-1,3,4-oxathiazolinone und ihre Verwendung gegen Endoparasiten sind bereits bekannt, ihre Wirkungen befriedigen jedoch nicht immer (DE-OS 2 604 110). Ferner sind 2-Aryloxy-1,3,4-oxadiazolinone vorbeschrieben (Pilgram, J. Heterocyclic Chem. 39, 823 (1982)); über ihre Verwendung zur Bekämpfung von Endoparasiten ist jedoch nichts bekannt.

1. Die vorliegende Erfindung betrifft substituierte 1,3,4-Oxa- und Thiadiazolinone der Formel (I)

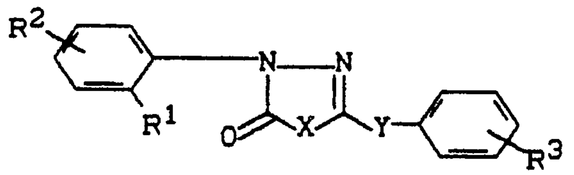

(I)

in welcher

$R^1$ für Wasserstoff, Alkyl, Alkoxy, Alkylthio, Halogen, Halogenalkyl, Halogenalkoxy, Halogenalkylthio steht,

$R^2$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,

$R^3$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, Alkylcarbonyl, Carbal koxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,

X für O oder S steht,

Y für O oder S steht,

ausgenommen die Verbindungen 5-Phenoxy-3-(3,4-di-chlorphenyl)-1,3,4-oxadiazol-2(3H)-on, 5-Phenoxy-3-(3-chlor-4-fluorphenyl )-1,3,4-oxadiazol-2-(3H)-on.

2. Verfahren zur Herstellung der neuen substiutierten 1,3,4-oxa- und Thiadiazolinone der Formel (I)

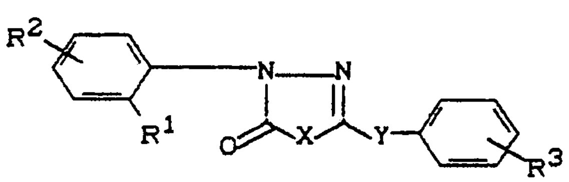

(I)

in welcher

$R^1$ für Wasserstoff, Alkyl, Alkoxy, Alkylthio, Halogen, Halogenalkyl, Halogenalkoxy, Halogenalkylthio steht,

$R^2$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dial kylsulfamoyl, Aryl, Aryloxy, Arylthio, die ihrerseits wieder substituiert sein können, steht,

$R^3$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,

X für O oder S steht,

Y für O oder S steht,

ausgenommen die Verbindungen 5-Phenoxy-3-(3,4-di-chlorphenyl)-1,3,4-oxadiazol-2(3H)-on, 5-Phenoxy-3-(3-Chlor-4-fluorphenyl)-1,3,4-oxadiazol-2-(3H)-on dadurch gekennzeichnet, daß man Verbindungen der Formel (II)

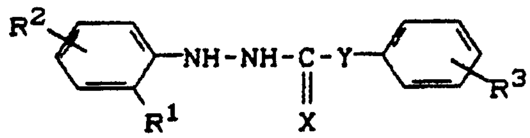

(II)

in welcher
X, Y, $R^1$, $R^2$, $R^3$ die oben angegebene Bedeutung haben, mit Carbonylierungsreagenzien wie Phosgen, Diphosgen oder Triphosgen umsetzt und die dabei erhaltenen Verbindungen der Formel III

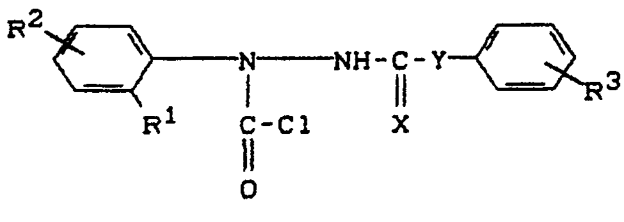

(III)

in welcher
X, Y, $R^1$, $R^2$, $R^3$ die oben angegebene Bedeutung haben
in Gegenwart von Basen cyclisiert.

Die Verbindungen der Formel I eignen sich hervorragend zur Bekämpfung von Endoparasiten, besonders auf dem Gebiet der Veterinärmedizin.

Bevorzugt sind Verbindungen der Formel I, in welcher

$R^1$ für Halogen, vorzugsweise Fluor, Chlor, Brom, Jod; für Alkyl mit vorzugsweise 1 bis 4, insbesondere 1 oder 2 Kohlenstoffatomen, wie Methyl, Ethyl, n.-und i.-Propyl und n.-, i.-, s.- und t.-Butyl; Alkoxy mit vorzugsweise 1 bis 4, insbesondere 1 oder 2 Kohlenstoffatomen, wie Methoxy, Ethoxy, n.-und i.-Propyloxy und n.-, i.-, s.- und t.-Butyloxy; Alkylthio mit vorzugsweise 1 bis 4, insbesondere 1 oder 2 Kohlenstoffatomen, wie Methylthio, Ethylthio, n.-und i.-Propylthio und n.-, i.-, s.- und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Fluor-, Chlorethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylthio, steht.

$R^2$ für Wasserstoff, Alkyl mit vorzugsweise 1 bis 4, insbesondere 1 oder 2 Kohlenstoffatomen, wie Methyl, Ethyl, n.-und i.-Propyl und n.-, i.-, s.-und t.-Butyl; Alkoxy mit vorzugsweise 1 bis 4, insbesondere 1 oder 2 Kohlenstoffatomen, wie Methoxy, Ethoxy, n.-und i.-Propyloxy und n.-, i.-, s.- und t.-Butyloxy; Alkylthio mit vorzugsweise 1 bis 4, insbesondere 1 oder 2 Kohlenstoffatomen, wie Methylthio, Ethylthio, n.-und i.-Propylthio und n.-, i.-, s.- und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Fluor-, Chlorethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylthio; im Falle von Phenyl, für Alkylendioxy mit vorzugsweise 1 oder 2 Kohlenstoffatomen wie Methylendioxy oder Ethylendioxy; im Falle von Phenyl für halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugsweise Fluor oder Chlor, insbesondere Fluor stehen wie Difluormethylendioxy, Trifluorethylendioxy, Tetrafluorethylendioxy. Weitere Substituenten sind Halogen, vorzugsweise Fluor, Chlor, Brom und Jod, insbesondere Chlor und Brom; Cyano; Nitro; Dialkylamino mit vorzugsweise 1 bis 4, insbesondere 1 oder 2

Kohlenstoffatomen je Alkylgruppe, wie Dimethylamino, Diethylamino, Methyl-n.-butylamino; Alkylcarbonyl mit vorzugsweise 2-4 Kohlenstoffatomen; Carbalkoxy mit vorzugsweise 2 bis 4, insbesondere 2 oder 3 Kohlenstoffatomen, wie Carbomethoxy und Carboethoxy; Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio, die ihrerseits wieder substituiert sein können, steht,

$R^3$ für Alkyl mit vorzugsweise 1 bis 4, insbesondere 1 oder 2 Kohlenstoffatomen, wie Methyl, Ethyl, n.-und i.-Propyl und n.-, i.-, s.- und t.-Butyl; Alkoxy mit vorzugsweise 1 bis 4, insbesondere 1 oder 2 Kohlenstoffatomen, wie Methoxy, Ethoxy, n.-und i.-Propyloxy und n.-, i.-, s.- und t.-Butyloxy; Alkylthio mit vorzugsweise 1 bis 4, insbesondere 1 oder 2 Kohlenstoffatomen, wie Methylthio, Ethylthio, n.-und i.-Propylthio und n.-, i.-, s.- und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogen atomen, wobei die Halogenatome gleich oder verschieden sind und als Halogenatome, vorzugsweise Fluor, Chlor oder Brom, insbesondere Fluor stehen, wie Trifluormethyl, Fluor-, Chlorethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylthio; im Falle von Phenyl, für Alkylendioxy mit vorzugsweise 1 oder 2 Kohlenstoffatomen wie Methylendioxy oder Ethylendioxy; im Falle von Phenyl für halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugsweise Fluor oder Chlor, insbesondere Fluor stehen wie Difluormethylendioxy, Trifluorethylendioxy, Tetrafluorethylendioxy. Weitere Substituenten sind Halogen, vorzugsweise Fluor, Chlor, Brom und Jod, insbesondere Chlor und Brom; Cyano; Nitro; Alkylcarbonyl mit vorzugsweise 2-4 Kohlenstoffatomen; Carbalkoxy mit vorzugsweise 2 bis 4, insbesondere 2 oder 3 Kohlenstoffatomen, wie Carbomethoxy und Carboethoxy; Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio, die ihrerseits wieder substituiert sein können, steht,

X für O oder S steht,

Y für O oder S steht.

Besonders bevorzugt sind Verbindungen der Formel I,

in welcher

$R^1$ für Halogen, insbesondere Chlor oder Fluor, $C_1$-$C_4$-Alkyl wie Methyl, Ethyl, $C_{1-4}$-Alkoxy wie Methoxy oder Ethoxy, $C_{1-4}$-Halogenalkoxy wie Trifluormethoxy, $C_{1-4}$-Halogenalkylthio wie Trifluormethylthio, steht,

$R^2$ für Halogen, insbesondere Chlor, Fluor oder Brom, $C_1$-$C_4$-Alkyl wie Methyl, Ethyl, $C_{1-4}$-Alkoxy wie Methoxy oder Ethoxy, $C_{1-4}$-Halogenalkoxy wie Trifluormethoxy, $C_{1-4}$-Halogenalkylthio wie Trifluormethylthio, Nitro, Cyano; Carbalkoxy wie Carbomethoxy und Carboethoxy; Alkylsulfonyl wie Methylsulfonyl und Ethylsulfonyl, steht,

$R^3$ für Halogen, insbesondere Chlor, Fluor oder Brom, $C_1$-$C_4$-Alkyl wie Methyl, Ethyl, $C_{1-4}$-Alkoxy wie Methoxy oder Ethoxy, $C_{1-4}$-Halogenalkoxy wie Trifluormethoxy, $C_{1-4}$-Halogenalkylthio wie Trifluormethylthio, Nitro, Cyano; Carbalkoxy wie Carbomethoxy und Carboethoxy; Alkylsulfonyl wie Methylsulfonyl und Ethylsulfonyl, steht,

X für O oder S steht,

Y für O oder S steht.

Ganz besonders bevorzugt sind Verbindungen der Formel I,

in welcher

$R^1$ für Halogen, insbesondere Fluor, Chlor, $NO_2$, $CH_3$, $OCH_3$, $CF_3$ steht

$R^2$ für Halogen, insbesondere Fluor, Chlor, Brom, $CH_3$, $OCH_3$, $NO_2$, CN steht,

$R^3$ für Halogen, insbesondere Fluor, Chlor, Brom, $CH_3$, $OCH_3$, $NO_2$, CN steht,

X für O steht,

Y für O steht.

Im einzelnen seien folgende Verbindungen der Formel (I) genannt, in welcher die Reste $R^1$, $R^2$, $R^3$, X und Y die angebene Bedeutung besitzen.

| $R^1$ | $R^2$ | $R^3$ | X | Y |
|---|---|---|---|---|
| -Cl | -H | -4-Cl | O | O |
| -Cl | 3-$CH_3$ | -H | O | O |
| -H | -4Br | -H | O | O |
| -$CH_3$ | -3-$CH_3$ | -H | O | O |
| -$CH_3$ | -3-$CH_3$ | -4-Cl | O | O |
| -$CH_3$ | 3-Cl | -H | O | O |
| -Cl | -4-Cl | -4-Cl | O | O |
| -Cl | -3-$CH_3$ | -4-Cl | O | O |
| -H | 3,4-$Cl_2$ | -H | O | O |
| -F | -5-F | -4-$CH_3$ | O | O |
| -F | -6-F | -H | O | O |
| -Cl | 4,6-$Cl_2$ | -H | O | O |
| -Br | -H | -H | O | O |
| -$OCH_3$ | -4-Cl | -4-Cl | O | O |
| -Cl | -H | 4-$CH_3$ | O | O |
| -$CH_3$ | -4-$CH_3$ | -H | O | O |
| -Cl | 6-Cl | -4-Cl | O | O |
| -2-$SCF_3$ | -H | -H | O | O |
| -$CH_3$ | -3,4-$CH_3$ | -4-Cl | O | O |
| -Cl | -H | -H | S | S |
| -H | 3,4-$Cl_2$ | -4-Cl | S | S |
| -$CH_3$ | -3-$CH_3$ | -H | S | S |
| -$CH_3$ | -3-$CH_3$ | -4-$CH_3$ | S | S |
| -Cl | -3-Cl | -H | S | S |
| -Cl | 6-Cl | -H | S | S |
| -F | -H | -H | S | S |
| -H | 4-Br | -4-Cl | S | S |
| -$OCH_3$ | -4-Cl | -H | O | S |
| -Cl | -3-Cl | -3-Cl | S | S |
| -F | -6-F | -H | O | S |
| -Br | -H | -H | O | S |
| -Cl | -3-$CH_3$ | -H | S | O |
| -Cl | -H | -H | S | O |
| -$CH_3$ | -3-$CH_3$ | -H | S | O |
| -Cl | -H | -4-Cl | S | O |
| -$CH_3$ | -3-$CH_3$ | -3-Cl | S | O |
| -F | 5-F | 4-Cl | S | O |
| -$CH_3$ | -$CH_3$ | 4-$CH_3$ | S | O |
| -Cl | -H | -4-$CH_3$ | S | O |
| -Cl | -4-$CH_3$ | -4-$CH_3$ | S | O |

Setzt man bei der Herstellung als Verbindung der Formel (II) 2-(2-Chlorphenyl)-hyrazincarbonsäurephenylester und als Carbonylierungsreagenz Phosgen ein, so läßt sich das Verfahren durch folgendes Formelschema darstellen:

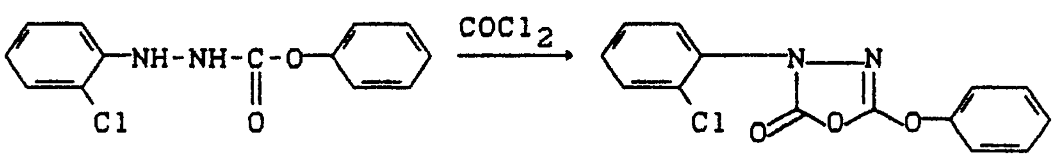


Bevorzugt werden Verbindungen der Formel II eingesetzt, in denen $R^1$, $R^2$, $R^3$, X, Y die bei den Verbindungen der Formel (I) angegebenen bevorzugten Bedeutungen besitzt.

Im einzelnen seien folgende Verbindungen der Formel (II) genannt:

2-(2-Chlorphenyl)-hydrazincarbonsäure-(4-chlorphenyl)ester, 2-(2,3-Dimethylphenyl)-hydrazincarbonsäurephenylester, 2-(2,5-Difluophenyl)-hydrazincarbonsäure-(4-bromphenyl)-ester, 2-(2-Chlor-3-methyl)-hydrazincarbonsäurephenylester, 2-(2,4-Dimethylphenyl)-hydrazincarbonsäure(4-chlorphenyl)ester, 2-(2-Chlorphenyl)-hydrazindithiocarbonsäure-phenylester, 2-(2,3-Dimethylphenyl)-hyrazindithiocarbonsäure-(4-chlorphenyl)-ester, 2-(4-Bromphenyl)-hydrazindithiocarbonsäure-(3-chlorphenyl)ester 2-(o-Tolyl)-hydrazindithiocarbonsäurephenylester, 2-(2-Chlorphenyl)-hydrazindithiocarbonsäure-(4-nitrophenylester, 2-(2-Chlorphenyl)-hydrazinthionocarbonsäurephenylester, 2-(2,3-Dimethylphenyl)-hydrazinthionocarbonsäure-(4-chlorphenylester), 2-Phenylhydrazincarbonsäure-phenylester, 2-(2,5-Difluorphenyl)hydrazincarbonsäure-p-tolylester, 2-(2-Methoxyphenyl)-hydrazinthiono-p-tolylester, 2-(2-Chlorphenyl)-hydrazincarbonsäurephenylthiolester, 2-(2,3-Dimethylphenyl)-hyrazincarbonsäure-(4-chlorphenyl)-thiolester, 2-(2,5-Difluorphenyl)-hydrazincarbonsäurephenylthiolester.

Die Verbindungen der Formel (II) sind teilweise bekannt oder lassen sich nach an sich bekannten Verfahren herstellen (Pilgram, J. Heterocyclic Chem. 39,823 (1982); US-P 3 395 234).

Die Umsetzung erfolgt bei Temperaturen von 20 - 200 °C, bevorzugt bei 50 - 150 °C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecen(DBU), 1,4-Diazabicyclo(2,2,2)octan (DABCO), Diazabicyclo(3,2,0)nonen (DBN), Ethyl-diisopropylamin.

Die Verbindungen der Formeln II und die Basen werden im Verhältnis 1:1 bis 1:1,5 zueinander eingesetzt. Bevorzugt ist ein etwa äquimolares Verhältnis.

Nach erfolgter Umsetzung wird das Verdünnungsmittel zum Teil (zu ca. 50 %) abdestilliert, der Rest mit wässriger Säure versetzt und die Verbindungen der Formel I in an sich bekannter Weise isoliert, indem sie mit einem geeigneten Lösungsmittel, z.B. Ether oder Methylenchlorid, extrahiert werden. Die Verbindungen der Formel I können anschließend in üblicher Weise, z.B. chromatographisch, gereinigt werden.

Die Wirkstoffe eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen- Sie sind dabei gegen alle oder einzelne Entwicklungsstadien der Schadlinge sowie gegen resistente und normal sensible Arten wirksame Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so das durch den Einsatz der Wirkstoffe eine wirtschaftlichere und einfaohere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln;

Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen be schrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß.Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure. Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zübereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungsund Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Starke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zübereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

## Beispiel A

In vivo Nematodentest

Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben

wurden oder so geschädigt sind, das sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 1 | 25 |
| 9 | 10 |

Beispiel B

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Würmer vor und nach der Behandlung quantitativ auszählt.

Ein völliges Sistieren der Eiausscheidung nach der Behandlung bedeutet, das die Würmer abgetrieben wurden oder so geschädigt sind, daß sie keine Eier mehr produzeiren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 1 | 5 |
| 8 | 5 |
| 9 | 25 |

## Herstellungsbeispiele

### Beispiel 1:

Herstellung von 5-(4-Chlorphenoxy)-3-(2-chlorphenyl)-1,3,4-oxadiazolin-2-on

Zu einer Lösung von 5 g (0,05 Mol) Phosgen in 50 ml Toluol gibt man bei Raumtemperatur 14,9 g (0,05 Mol) 2-(2-Chlorphenyl)-hydrazincarbonsäure-(4-chlorhpenyl)ester und erhitzt die erhaltene Lösung langsam auf 100 °C, wobei mit einem Trockeneiskühler die entwickelnden Dämpfe kondensiert werden. Nach Erreichen der Siedetemperatur wird durch einen Schlangenkühler ausgetauscht und die Reaktionsmischung bis zum Ende der Gasentwicklung am Rückfluß gekocht. Unter reduziertem Druck engt man nun die Lösung ein und löst den erhaltenen Rückstand in 100 ml Methylenchlorid. Zu dieser Lösung werden 5 g (0,05 Mol) Triethylamin gegeben und 12h bei Raumtemperatur nachgerührt. Zur Auflösung des ausgeschiedenen Triethylaminhydrochlorids wird mit 100 ml Wasser verrührt, die organische Schicht abgetrennt und nacheinander mit verdünnter Salzsäure und Wasser gewaschen. Nach dem Trocknen ($Na_2SO_4$) wird im

Vakuum unter reduziertem Druck zur Trockne eingedampft und der Rückstgand aus Diisopropylether umkristallisiert. Ausbeute: 81 % der Theorie, Schmelzpunkt 110 °C.

**Analog Beispiel 1 werden die folgenden Verbindungen hergestellt:**

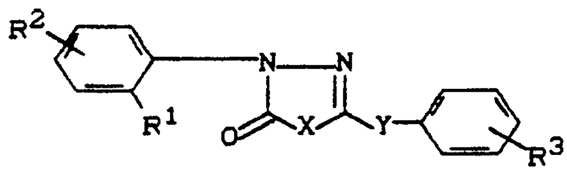

| Bsp. Nr. | $R^1$ | $R^2$ | $R^3$ | X | Y | Schmp. |
|---|---|---|---|---|---|---|
| 2 | H | 4-Br | H | O | O | 113° C |
| 3 | H | 3-Cl | H | O | O | 76° C |
| 4 | H | 4-Cl | 4-Cl | O | O | 112° C |
| 5 | H | 3-Cl | 4-Cl | O | O | 106° C |
| 6 | H | 4-Br | 4-Cl | O | O | 141° C |
| 7 | Cl | H | 4-Br | O | O | 106° C |
| 8 | $CH_3$ | 3-$CH_3$ | 4-Cl | O | O | 101° C |
| 9 | F | 5-F | 4-Cl | O | O | 116° C |
| 10 | H | 4-Cl | 4-Br | O | O | 122° C |
| 11 | -Cl | -H | -4-$CH_3$ | O | O | 72° C |
| 12 | -H | 3,5-$(CH_3)_2$ | -H | O | O | 103° C |
| 13 | -H | 3 Cl, 4 F | -H | O | O | 96° C |
| 14 | -H | 3,5-$(CH_3)_2$ | -4 Cl | O | O | 123° C |

## Ansprüche

1. Substituierte 1,3,4-Oxa- und Thiadiazolinone der Formel (I)

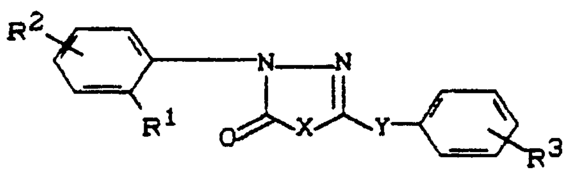

(I)

in welcher
$R^1$ für Wasserstoff, Alkyl, Alkoxy, Alkylthio, Halogen, Halogenalkyl steht,
$R^2$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^3$ für einen oder mehrere, gleiche oder verschiedene der Reste Wasserstoff, Alkyl, Alkoxy, Al kylthio, Halogenalkyl, Halogenalkoxy, Halogenalkylthio, Alkylendioxy, Halogenalkylendioxy, Halogen, CN, $NO_2$, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
X für O oder S steht,
Y für O oder S steht,
ausgenommen die Verbindungen 5-Phenoxy-3-(3,4-dichlorphenyl)-1,3,4-oxadiazol-2-(3H)-on, 5-Phenoxy-3-(3-chlor-4-fluorphenyl)-1,3,4-oxadiazolo-2-(3H)-on.

2. Verfahren zur Herstellung der 1,3,4-Oxa- und Thiadiazolinonen der Formel I

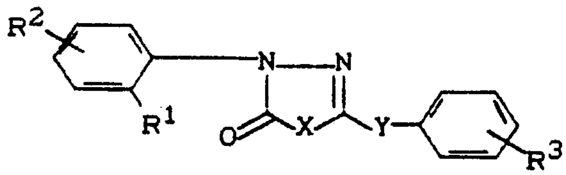

(I)

in welcher
$R^1$ für Wasserstoff, Alkyl, Alkoxy, Alkylthio, Halogen, Halogenalkyl steht,
$R^2$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_{29}$ $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^3$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
X für O oder S steht,
Y für O oder S steht,
ausgenommen die Verbindungen 5-Phenoxy-3-(3,4-dichlorphenyl)-1,3,4-oxadiazol-2-(3H)-on, 5-Phenoxy-3-(3-chlor-4-fluorphenyl)-1,3,4-oxadiazolo-2-(3H)-on,
dadurch gekennzeichnet, das man Verbindungen der Formel (II)

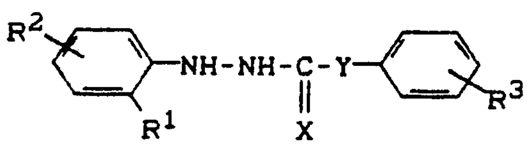

(II)

in welcher
X, Y, $R^1$, $R^2$, $R^3$ die oben angegebene Bedeutung haben,
mit Carbonylierungsreagenzien in Gegenwart von Basen umsetzt.

3. Verwendung von substituierten 1,3,4-Oxa- und Thiadiazolinone der Formel (I)

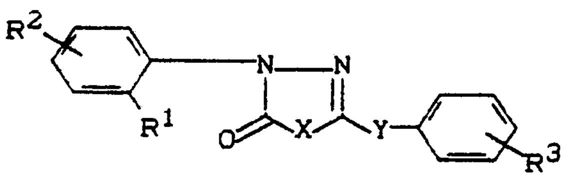

(I)

in welcher
$R^1$ für Wasserstoff, Alkyl, Alkoxy, Alkylthio, Halogen, Halogenalkyl steht,
$R^2$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^3$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
X für O oder S steht,
Y für O oder S steht,
zur Bekämpfung von Endoparasiten.

4. Endoparasitizide Mittel, gekennzeichnet durch einen Gehalt an mindestens einem substiutierten 1,3,4-Oxa- oder Thiadiazolinon der Formel (I) gemäß Anspruch 3.

5. Verfahren zur Herstellung von endoparasitiziden Mitteln, dadurch gekennzeichnet, daß man 1,3,4-Oxa- oder Thiadiazolinone der Formel (I) gemäß Anspruch 3 mit Streckmitteln und/oder oberflächenaktiven Mitteln vermischt.

6. Verwendung von 1,3,4-Oxa- und 1,3,4-Thiadiazolinonen der Formel (I) gemäß Anspruch 3 zur Herstellung von endoparasitiziden Mitteln.